Europäisches Patentamt

European Patent Office

Office européen des brevets

(19)

(11) Veröffentlichungsnummer: **0 149 468**
**B1**

(12) **EUROPÄISCHE PATENTSCHRIFT**

(45) Veröffentlichungstag der Patentschrift: **31.10.90**

(21) Anmeldenummer: **85100179.2**

(22) Anmeldetag: **10.01.85**

(51) Int. Cl.⁵: **A 61 K 37/24,** A 61 K 37/02, A 61 K 39/39, G 01 N 33/50

(54) **Biologisch wirksame Substanz mit hormonellen Eigenschaften, Verfahren zu ihrer Herstellung und Verwendung von Histonen für medizinische Zwecke.**

(30) Priorität: **12.01.84 DE 3400928**
**16.02.84 DE 3405620**

(43) Veröffentlichungstag der Anmeldung:
**24.07.85 Patentblatt 85/30**

(45) Bekanntmachung des Hinweises auf die Patenterteilung:
**31.10.90 Patentblatt 90/44**

(84) Benannte Vertragsstaaten:
**AT BE CH DE FR GB IT LI LU NL SE**

(56) Entgegenhaltungen:
**EP-A-0 101 063      FR-A-2 305 191**
**DE-A-2 732 587      FR-A-2 315 943**
**FR-A-2 265 404**

**BIOLOGICAL ABSTRACTS, Band 73, Nr. 12, (1982) Zusammenfassung 80609 Philadelphia, P.A., US; BUSCH et al.: "Ubiquitin-protein conjugates"**

**Die Akte enthält technische Angaben, die nach dem Eingang der Anmeldung eingereicht wurden und die nicht in dieser Patentschrift enthalten sind.**

(73) Patentinhaber: **SYMBIOTEC Gesellschaft für Forschung und Entwicklung auf dem Gebiet der Biotechnologie GmbH**
**Kornmarkt 34**
**D-6348 Herborn (DE)**

(72) Erfinder: **Rusch, Volker, Dipl. Biol. Dr. rer. nat.**
**Schwalbenweg 6**
**D-6348 Herborn (DE)**
Erfinder: **Reichhart, Robert, Dipl.-Chem.**
**Uhlandstrasse 15**
**D-6650 Homburg/Saar (DE)**
Erfinder: **Zeppezauer, Michael, Prof. Dr. rer. nat.**
**Eichendorfferstrasse 13**
**D-6601 Saarbrücken-Scheidt (DE)**
Erfinder: **Jörnvall, Hans, Prof. Dr.**
**Ursviksvagen 109B**
**S-17246 Sundbyberg (SE)**

(74) Vertreter: **Pätzold, Herbert, Dr.-Ing.**
**Steubstrasse 10**
**D-8032 Gräfelfing/München (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es sind zehlreiche verschiedene Thymus-Präparationen bekannt. Hierzu gehört auch die Thymus-Präparation (HTH) nach Comsa & Bernardi ("Extraction, Fractionation und Testing of a Homogenous Thymic Hormone Preparation", veröffentlicht in Annals of the New York Academy of Sciences, Vol. 240, pages 402—403, Febr. 28, 1975).

Comsa konnte unter anderem zeigen, daß HTH das Immunsystem Immunstimulierende und endokrinologisch wirksame Eigenschaften besitzt. Er ermutete, daß diese Wirkung einem speziellen Thymus-Hormon zuzuordnen ist ("Hormonal Coordination of the Immune Response", Vol. 92, Springer-Verlag, 1982). Comsa hat auch nachgewiesen, daß Thymus-Präparationen aufgrund ungeklärter Nebenwirkungen von nach nicht definierten Substanzen therapeutisch unwirksam oder abträglich sein können. Es können also in Thymus-Präparationen Fremdsubstanzen verhanden sein, die die wirksamen Eigenschaften des vermuteten Thymus-Hormone innerhalb der Thymus-Präparation Überdecken oder hemmen bzw. stören und die für den Patienten ein beträchtliches Risiko darstellen können.

Aufgabe der Erfindung ist es, eine biologisch wirksame Substanz der vorstehenden Art anzugeben, die einen Eindeutigen Bezug zur gewünschten thymushormonartigen Wirkung aufweist, also im wesentlichen frei von Fremdsubstanzen ist, die diese Wirkung negativ beeinträchtigen könnes. Damit wird vor allem auch das Ziel verfolgt, erstmals die Standardisierung einer solchen Präparat-Wirkung ermöglichen zu können und damit auch Wege für Herstellungsverfahren der Wirksubstanz aus biologischen Ausgangsmaterialien und die gezielte Verwendung solcher Substanzen zu eröffnen, die zu weitgehend reinen bzw. Fremdsubstanzfreien Präparaten mit hormonähnlichen Wirkungen führen.

Die Aufgabe wird durch die in den Ansprüchen bezeichnete Erfindung gelöst.

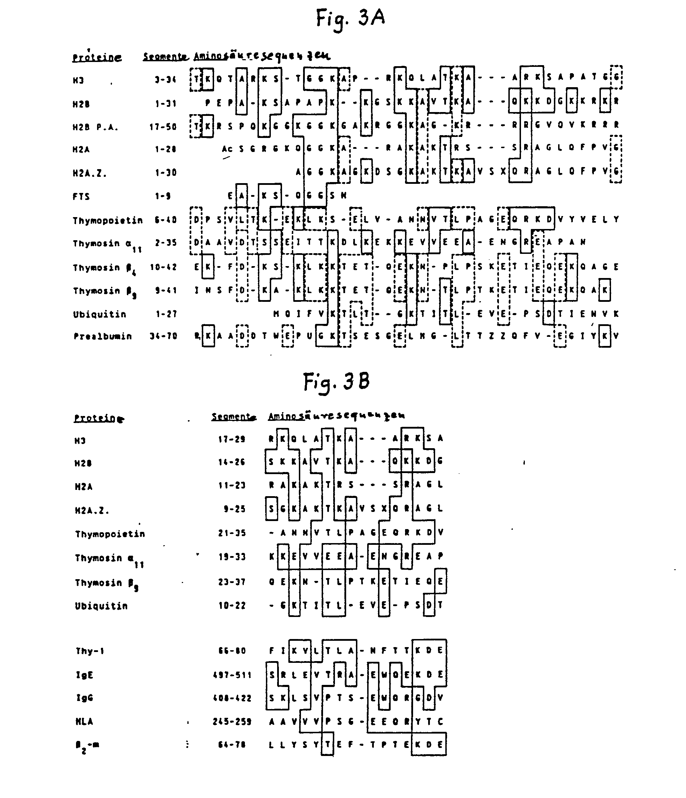

Es war für die Erfinder überraschend, daß sie in der HTH-Thymus-Fraktion nach Comsa-Bernardi kein spezifisches Thymus-Hormon lokalisieren konnten, das eindeutig immunologische und endokrinologische Eigenschaften besitzt. Bei Untersuchungen an HTH ließen sich als hormonelle Wirksubstanzen zwei reproduzierbar zu detektierende Polypeptidketten $HTH_\alpha$ und $HTH_\beta$ darstellen, deren Aminosäuresequenzen sich überraschenderweise als identisch mit Sequenzen des H2A- und H2B-Histons erwiesen. Es konnte erfinderseits damit erstmals gezeigt werden, daß Histone, die bisher nur als Zellkernproteine bekannt waren, hormonähnliche Eigenschaften mit extrazellulären und endokrinen Aktivitäten besitzen müssen. Histon/Thymushormon-Strukturvergleiche zeigen, daß die hormonähnlichen, insbesondere thymushormonartigen Eigenschaften der Histone zumindest im wesentlichen durch die evolutionär variablen Teile der Histone bestimmt sind (nachfolgende Beschreibung und Fig. 3 (A)) und sich nicht in den evolutionär invariablen (konstanten) Histonteilen finden lassen. Die Erfinder konnten durch Strukturvergleiche in den evolutionär variablen Histonabschnitten der H2-Histone Teilsequenzen mit Hormonwirkung (aktive Histonsequenzen) lokalisieren (nachfolgende Beschreibung und Fig. 3(B)), die mit Thymushormonen Ähnlichkeiten aufweisen. Dabei konnte auch gezeigt werden, daß Ähnlichkeiten insbesondere zu N-terminalen Histonbereichen bestehen und Wechselbeziebungen zwischen Histonen und Proteinen des Immunsystems, insbesondere Ubiquitin, verhanden sind.

Die Erfindung offenbart damit, daß Histone, insbesondere H2A- und H2B-Histone und bestimmte Teile hiervon, hormonähnliche, insbesondere thymushormonartige Eigenschaften besitzen und nicht nur Zellkernbausteine sind. Es zeigte sich außerdem, daß die das Immunsystem immunstimulierenden und/oder die endokrinologisch wirksamen Substanzen, die in den verschiedensten bekannten Thymus-Präparationen enthalten sind, Ähnlichkeiten mit den evolutionär variablen Abschnitten der Histone aufweisen und damit möglicherweise unter einer Histon-Familie subsummierbar sind, die hinsichtlich ihrer neu entdeckten Hormon-Eigenschaften gegebenenfalls neu zu definieren sein wird.

Die Erfindung offenbart damit relativ einfache Wege zur gezielten Herstellung von des Immunsystem immunstimulierenden und/oder endokrinologisch wirksamen Präparaten mit erstmal standardisierbarer Präparat-Wirkung. So ist es nicht mehr notwendig, allein aus Thymus-Präparaten bestimmte Peptide mit der gewünschten Wirkung zu isolieren, es können auch Präparate aus anderen biologischen Ausgangsmaterialien hergestellt werden, die möglichst reich an isolierbaren Histonen, insbesondere H2A- und H2B-Histonen sind.

Da es inzwischen bekannt ist, daß H2A- und H2B-Kalbshistone identisch sind mit menschlichen H2A- und H2B-Histonen, ist es völlig unbedenklich, erfindungsgemäße Präparate für die Humanmedizin z.B. aus Kelbsthymus oder einem anderen geeigneten endokrinen Organ des Kalbes bzw. des Rindes horzustellen, das reich an H2A- und H2B-Histonen ist.

Für eine wirtschaftliche Herstellung des erfindungsgemäßen Präparates auf synthetischem Wege ist die Erkenntnis wichtig, daß die das Immunsystem immunstimulierenden und/oder endokrinologisch aktiven Eigenschaften der Histone innerhalb der evolutionär variablen Abschnitte der Histone liegen. Es genügt daher, lediglich die evolutionär variablen Abschnitte der betreffenden Histone oder Teile hiervon zu synthetisieren, die die aktive Sequenz enthalten. Das gleiche glit auch für eine gentechnologische Herstellung bzw. für eine Kombination dieser Herstellungsverfahren.

Die Erfindung ist daher nicht auf die Verwendung von Histonen, insbesondere H2A- und H2B-Histonen als immunstimulierende bzw. endokrinologisch wirksame Präparate beschränkt. Es können auch nur die evolutionär variablen Abschnitte bzw. die wirkungsmäßig aktiven Bereiche dieser variablen Abschnitts oder eine Kombination aus einem oder mehreren solcher Abschnitte mit einem oder mehreren Histonen verwendet werden.

Schließlich ist die Erfindung nicht auf die Verwendung von Histonen oder Histonabschnitten mit hormonähnlichen Eigenschaften beschränkt. So kann die Verwendung von solchen Histonen oder Histonabschnitten mit wenigstens einem Protein des Immunsystems, insbesondere Ubiquitin, erfindungsgemäß besondere vorteilhaft sein.

Erfindungsgemäß ist der Begriff "Histone" aufgrund ihrer neu entdeckten hormonellen Eigenschaften weiter zu verstehen als im herkömmlichen Sinne, als man unter Histonen nur Zellkernbauteile verstand.

Die erfinderseits erkannte Histon-Wirkung als immunstimulierende Substanz und als Synergist und/oder Antagonist von endokrinen Drüsen (endokrinologische Wirksamkeit) setzt voraus, daß die Histone genz oder wenigstens in Form von Teilsequenzen, die für die neu erkannte Wirkung verantwortlich sind, in der Blutbahn und/oder im Lymphsystem zirkulieren, um ihre hormonelle Wirkung entfelten zu können. Eine derartige Wirkung war nach der bisherigen Definition der Histone nicht zu erwarten.

Die der Erfindung zugrunde liegenden Erkenntnisse über neue Eigenschaften der Histone und die im Zusammenhang hiermit stehenden Untersuchungen an dem homeostatischen Thymushormon (HTH) werden nachstehend näher beschrieben. Die in Klammern gesetzten Ziffern verweisen auf Literaturstellen des Literaturverzeichnisses am Ende der Beschreibung, der außerdem Figuren angefügt sind, die die Untersuchungsergebnisse verdeutlichen. In den Figuren zeigt:

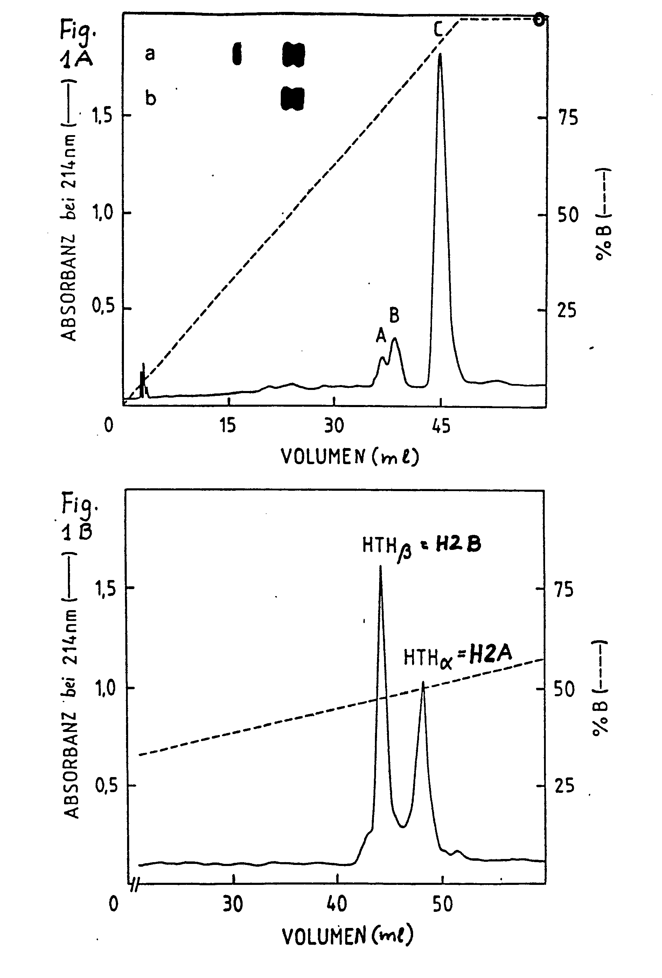

Fig. 1(A) die Reinigung der HTH-Präparation nach Bernardi und Comsa (2) mit Hilfe von HPLC an einer µ Bondapak-C18-Säule in 0,1% Trifluoressigsäure unter Verwendung von Isopropanolgradienten (%B).

Innerhalb der Fig. 1(A) ist eine SDS-Polyacrylamidgel-Elektrophorese von (a) HTH-Ausgangsmaterial und (b) Komponenten $HTH_\alpha$ und $HTH_\beta$ der HTH-Fraktion C dargestellt;

Fig. 1(B) die Aufteilung der HTH-Fraktion C (Fig. 1(A)) in die zwei Komponenten $HTH_\alpha$ und $HTH_\beta$ mit Hilfe von HPLC an einer µ Bondapak-C18-Säule in 0,1% Trifluoressigsäure unter Verwendung von Acetonitrilgradienten (%B);

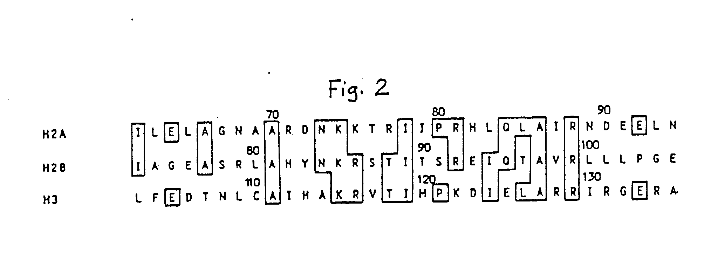

Fig. 2 die Ausrichtung der C-terminalen Regionen der Histone H2A, H2B und H3. Die Histone H2A und H2B stammen vom Kalb oder vom Menschen. (Kalbs- und menschliche H2-Histone sind identisch). Das H3-Histon stammt vom Kalb. Es sind die H2A-Sequenzen 62—94, die H2B-Sequenzen 73—105 und die H3-Sequenzen 103—135 (das C-terminale Ende) einander gegenübergestellt. Identische Aminosäuren sind zwischen Linien eingeschlossen;

Fig. 3(A) Vergleiche von Aminosäure-Sequenzen einer ersten Gruppe von Histonen mit einer zweiten Gruppe von Thymushormonen: Histone H3 (vom Kalb (37)), H2B (vom Kalb und vom Menschen (37)), H2B P. A. (Seeigel-Sperma Parechinus angulosus (37)), H2A (vom Kalb und vom Menschen (37)) und H2A.Z (Variante vom Kalb (28)). FTS bezeichnet ein Thymusserum (vom Schwein (14)). Thymopoietin (vom Ochsen (13)), Thymosine $\alpha_{11}$, $\beta_4$ und $\beta_9$ (vom Kalb (10—12)), Ubiquitin (vom Ochsen (16)) und Prealbumin (vom Menschen (17)). Identische Aminosäuren zwischen den beiden Gruppen sind zwischen ununterbrochenen Linien eingeschlossen. Aminosäuren, die nur innerhalb einer der beiden Gruppen identisch sind, sind zwischen gestrichelte Linien eingeschlossen. Sequenz- Unterbrechungen (gaps) sind vergenommen worden, um die maximalen strukturellen Übereinstimmungen zu verdeutlichen.

In Fig. 3(A) sind im wesentlichen die evolutionär variablen Histonabschnitte der H2A- und H2B-Histone erfaßt (H2A etwa die Aminosäuren 1 bis 36, H2B etwa die Aminosäuren 1 bis 31). Innerhalb dieser Abschnitte liegen die aktiven Sequenzen, die für die Erfindung besondere wesentlich sind;

Fig. 3(B) Weitere Vergleiche von Aminosäuresequenzen der zwei Proteingruppen in Fig. 3(A) mit Zelloberflächen-Antigen (von der Ratte (Thy-1)), Immunoglobulin (vom Menschen bzw. Kaninchen (IgE, IgG)), Histokompaktibilitäts-Antigen (vom Menschen ($\beta_2$-m)). Identische Aminosäuren befinden sich zwischen ununterbrochenen Linien.

Die Fig. 3(B) zeigt u.a. vom H2A und H2B Abschnitte innerhalb der evolutionär variablen Histonabschnitte (H2A Aminosäuren 11 bis 23, H2B Aminosäuren 14 bis 26), die zur weiteren Lokalisierung der aktiven Sequenzen erfindungsgemäß wichtig sein können.

Verschiedene Funktionen des Thymus sind bereits eindeutig nachgewiesen worden: Die Proliferation und Differenzierung der lymphoiden Vorläuferzellen sowie die Produktion und Ausscheidung von verschiedenen Thymushormonen. Eines dieser Hormone, des homeostatische Thymushormon (HTH), wurde, wie eingangs schon gesagt, schon früh von Comsa und Mitarbeitern (1, 2) entdeckt. Über das HTH wurde berichtet, daß es vollständig die folgen der Thymektomie unterdrückt (3), daß es die Antikörperproduktion in thymektomierten Tieren wiederherstellt (4) und daß es einen chemotaktischen Einfluß auf Lymphozyten ausübt (5). Auch konnte ein regulatorischer Einfluß des HTH auf andere endokrine Drüsen gezeigt werden (6, 7). So ist HTH Antagonist des Thyroxin (6), des Adrenocorticotropen Hormons (ACTH), des Desoxycorticosterons, des Thyrotropine (TSH) und der Gonadotropine. HTH ist Synergist des Wachstumshormone (6, 7). Zusätzlich wurde noch ein radioprotektiver Einfluß des HTH vorgeschlagen (8).

HTH wurde im Thymus, in den Lymphknoten und in der Milz von normalen Ratten vor der Pubertät gefunden. Es wurde berichtet, daß es drei Tage nach Thymektomie vollständig aus den Lymphknoten und der Milz verschwindet (5, 6). Aus menschlichem Urin konnte eine dem HTH gleichende Fraktion isoliert werden, die die folgen der Thymektomie bei Meerschweinschen verhinderte. Diese den Thymus ersetzende Fraktion fehlte im Urin eines Mädchens, dem früher der Thymus vollständig entfernt worden war (5). Somit beweisen vorschiedene Tatsachen, daß das HTH sekretiert, im Körper verteilt und ausgeschieben wird.

Die Struktur des HTH konnte bisher noch nicht aufgeklärt werden. Dagegen wurden schon einige andere Polypeptide des Thymus charakteriziert, und zwar mehrere Thymosine (9—12). Thymopoietine (13) und das kleine Nonapeptid "serum thymic factor" (FTS) (14). Nach Isolierung aus dem Thymus wurde ebenfalls die Struktur des Proteins Ubiquitin bestimmt, dessen wahrscheinlicher Einfluß auf die Regulation des Proteinabbaus jetzt bekannt ist (15). Wieterhin wurde auch die Identität des "Serum-Thymushormons" mit Serumprealbumin aufgezeigt (17), das mögliche Verwandschaften zu gastrointestinalen Hormen besitzt (18).

Bei Berücksichtigung der verschiedenen Größen der Thymushormone, die von kleinen Polypeptiden bis zu dem Lymphozytenstimulierenden Hormon (LSH$_r$, M$_r$ 80000) (19) und anderen großen Proteinen reichen, scheint es möglich zu sein, daß einige Katten Fragmente von größeren Proteinen sind. Diese Vermutung wird durch das kürzlich gefundene Thymosin $\alpha_{11}$ gestärkt, das sieben Aminosäuren zusätzlich zu denen des Thymosin $\alpha_1$ enthält, und durch das Prothymosin mit etwa 112 Aminosäuren, das die Sequenz das Thymosin $\alpha_1$ in seinem Aminoterminus enthält (20). Weiterhin ist eine Lys-Arg-Struktur, die ein typisches Signal für die Prohormonspaltung ist (21, 22), am C-terminalen Ende aller Thymopoietine vorhanden (13). Zusammenfassend kann gesagt werden, daß außer HTH mehrere Thymushormone charakterisiert worden sind. Spaltungen der Proformen scheinen wichtig zu sein, jedoch wurden bisher nur wenige Funktionen und keine strukturellen Verwandschaften nachgewiesen.

Nachstehend wird die weitgehende Reinigung und Ausbeute von zwei Komponenten, HTH$_\alpha$ und HTH$_\beta$ beschrieben, die sich erfinderseits als die einzigen reproduzierbar zu detektierenden Polypeptidketten der HTH-Präparate darstellten Die Aminosäuresequenzen des HTH$_\alpha$ und des HTH$_\beta$, deren Bestimmung erfinderseits separat beschrieben ist (23), sind erfinderseits als identisch mit den Sequenzen der Histone H2A und H2B erkannt werden. Da bisher keine extrazellulären oder endokrinen Aktivitäten von Histonen bekannt geworden sind, wurde erfinderseits die Möglichkeit untersucht, ob H2A und H2B bloße Verunreinigungen in den HTH-Präparaten sind. Es zeigte sich jedoch, daß HTH$_\alpha$ und HTH$_\beta$ die einzigen Komponenten sind, deren Anreicherung in HTH-Präparaten möglich war. Im Falle, daß die beiden Komponenten nicht selbst das ·HTH dargestellt hätten, hätte die HTH-Aktivität von einer Komponente stemmen müssen, die nur in geringsten Mengen hätte verhanden sein dürfen und die zusammen mit den Histonen hätte gereinigt werden müssen. Wegen dieser Möglichkeit wurde erfinderseits eine eingehende Prüfung der HTH$_\alpha$/H2A- und HTH$_\beta$/H2B-Strukturen durchgeführt, und zwar vor allem in Beziehung zu möglichen Spaltsignalen und Ähnlichkeiten mit anderen Thymoshormonen. Aufgrund dieser Beziehungen zeigten sich neue und überraschende Histon-Funktionen, die auch erklären, wie HTH und vielleicht andere Thymushormone eventuell in Verbindung mit Histonen und Ubiquitin in einem regulatorischen System zusammenwirken und die den Weg zu erfindungsgemäßen Verwendungen von Histonen in Medikamenten erst eröffneten.

Reinigung von HTH (Fig. 1(A) und 1(B))

Das Homeostatische Thymushormon HTH wurde aus Kalbsthymus unter Verwendung einer fünfstufigen Reinigungsmethode isoliert, die, wie in (1) beschrieben, aus Ausfällungen eines sauren Extrakts (1 M $H_2SO_4$) mit Ammoniumsulfat und Ethanol und folgenden chromatographischen Auftrennungen über Sephadex G-100, Hydroxyapatit und Sephadex G-25 besteht (2). Die Reinigung Wurde behindert durch proteolytische Einflüsse, bis ein Enzyminhibitor zugefügt wurde. Diese Fraktionen waren aktiv in den in der vorstehenden Einleitung beschriebenen biologischen Tests. Die endgultige Reinigung von zwei Komponenten mit M$_r$ 15000 und 16500 wurde mit Hilfe von HPLC en einem Waters-Gerät mit einer µBondapak C18-Säule unter Verwendung von Isopropanol- oder Acetonitrilgradienten in 0,1% Trifluoressigsäure durchgeführt.

Aminosäuresequenzanalyse

HTH wurde mit CNBr und mit proteolytischen Enzymen gespalten (23). Die Peptidgemische wurden über Sephadex G-50 fine in 30% Essigsäure vorfraktioniert und für die intakten HTH-Komponenten, wie oben beschrieben, durch HPLC weiter gereinigt.

Zur manuellen Sequenzanalyse wurde die Dimethylaminoazobenzol-Methode angewandt. Der Flüssigphasen-Sequenzabbau wurde mit einem Beckman 890D-Gerät unter Anwendung eines 0,1 M Quadrol-Peptidprogramms in Anwesenheit von mit Glycin vorbehandeltem Polybrene durchgeführt. Die Phenylthiohydantoine wurden mittels HPLC identifiziert (23).

Sequenzvergleiche

Die für HTH$_\alpha$/H2A und HTH$_\beta$/H2B bestimmten Aminosäuresequenzen wurden mit denen des Histone H3 (24, 25), der Thymosine (9—12), der Thymopoietine (13), des FTS (14), des Ubiquitins (16) und des Prealbumins (26) verglichen. Dabei wurde ein Computerprogramm unter Verwendung verschiedener Abschnittsgrößen zur Detektion von Ähnlichkeiten benutzt, unabhängig von Sequenzlücken (gaps) und unter Anwendung von Randomstrukturen mit derselben Zusammensetzung, um so den Grad von zufällig ähnlichen Strukturen (27) abschätzen zu können.

Resultate der Reinigung von HTH

Bei der Isolierung ohne Proteolyse enthielt des biologisch aktive HTH-Präparat von Bernardi und Comsa (2) keine kleinen Peptide. Bei der SDS-Polyacrylamidgel-Elektrophorese in einem 7,5% Gel unter reduzierenden und nichtreduzierenden Bedingungen ergab dieses Präparat jeweils zwei konstant vorhandene Peptidbanden entsprechend $M_r$ 15000 und $M_r$ 16500 (Fig. 1(A)). Die Molekulargewichtsbestimmung des nativen HTH durch Ausschlußchromatographie über Sephadex ergab einen Wert von etwa 30000, was eine dimere Form für das native HTH vermuten läßt. Im Gegensatz zu Berichten, daß HTH ein Glykopropein sein könnte (5) und mit Trifluoressigsäure (1) nicht fällbar sei, werden erfinderseite zum einen keine Kohlenhydrate und zum anderen ein Protein gefunden, des mit Trifluoressigsäure fällbar ist.

Um die zwei Hauptpolypeptidketten von kleineren und variierenden Verunreinigungen zu trennen, wurden die HTH-Präparate mittels HPLC unter Verwendung eines Isopropanol-Gradienten weiter aufgetrennt. Die zwei Komponenten wurden in einem Hauptpeak eluiert, der in Fig. 1(A) mit C bezeichnet ist. Diesem gehen zwei kleinere Peake (A und B) voraus. Letztere entsprechen mehreren Komponenten in variablen Mengen und mit elektrophoretischen Positionen (Fig. 1(A)), die identisch sind den inaktiven Komponenten aus den vorangehenden Reinigungsstufen (Die SDS-Polyacrylamidgel-Elektrophorese der Proben aus der Fraktion C unter reduzierenden und nichtreduzierenden Bedingungen bestätigte das Vorhandensein von zwei Peptidhauptkomponenten (Fig. 1(A)). Die Endgruppenbestimmung mit der Dansyl-Methode zeigte jedoch nur Prolin als N-terminale Aminosäure (die zweite Polypeptidkeite hat einen blockierten N-terminus). HPLC unter Verwendung eines weichen Acetonitrilgradienten ergab die Auftrennung der Fraktion C in die zwei Komponenten $HTH_\alpha$ und $HTH_\beta$ in reiner Form (Fig. 1(B)), wie durch SDS-Polyacrylamidgel-Elektrophorese (Fig. 1(A)) bestätigt wurde. Die Aminosäuresequenzen der zwei Proteine wurden durch Sequenzabbau in Verbindung mit Fragmentierung durch CNBr und proteolytische Enzyme, wie in (23) beschrieben, bestimmt. Sie zeigten, daß $HTH_\alpha$ identisch dem Histon H2A und $HTH_\beta$ identisch dem Histon H2B sind. Es wurden erfinderseits keine Anzeichen für eine Modifizierung von Aminosäuren im HTH beobachtet (23).

Vergleich mit anderen Sequenzen (Fig. 3(A), 3(B))

Die Strukturen von $HTH_\alpha$/H2A und $HTH_\beta$/H2B wurden auf Ähnlichkeiten hin mit den Thymosinen $\alpha_1$, $\alpha_{11}$, $\beta_4$, $\beta_9$ (9—12), den Thymopoietinen (13), den Histonen H2A, H2B, H3 und dem Untertyp H2A.Z (28), Übiquitin (16) und Prealbumin (26) untersucht, um Proteine zu erfassen, die funktionell entweder den Thymushormonen oder den Histonen verwandt sind. Ein Computerprogramm unter Verwendung variabler Fragmentgrößen (27) zeigte, daß keine auffellenden Ähnlichkeiten existieren, und daß keines der jeweils verglichenen Paare für sich allein bedeutende Ähnlichkeiten besitzt. Sieben der insgesamt besten fünfzehn Fits (solche mit 6 und 7 identischen Aminosäuren pro 15 Aminosäuren) zwischen H2A-H2B, H2A-H3 und H2B-H3 richten jedoch, unabhängig voneinander, die C-terminalen Regionen dieser Histone so untereinander aus (Fig. 2), daß eine nöchstmögliche Übereinstimmung erhalten wird.

Versuche eines direkten Vergleichs in den N-terminalen Bereichen der Histon/HTH-Strukturen ergeben weniger identitäten bei paarweisen Vergleichen (5 oder weniger pro 15 Aminosäuren). Bei der Einführung von Sequenzunterbrechungen (gaps) kann jedoch die Homologie zwichen den verschiedenen Vergleichspaaren erhöht werden. Der N-terminale Vergleichsbereich, der eine maximale Zahl von identischen Gruppen und eine minimale Zahl von gaps enthält, ergibt frei andere unabhängige Übereinstimmungen. Dieser Bereich ist in Fig. 3(A) gezeigt. Eine weitere Übereinstimmung besteht darin, daß dieser Teil mit nur wenigen Sequenzverschiebungen auch Ähnlichkeiten mit den untereinander ähnlichen C-terminalen Bereichen der Histone selbst zeigt (Fig. 2). Weiterhin überdeckt diese Region (Fig. 3(A)) auch die Bereiche, die an Bindungswechselwirkungen (29) beteiligt sind (Fig. 3(B)). Folglich lessen sich unabhängig voneinander vorgeschlagene Funktionelle Ähnlichkeiten jetzt mit offenbar vorhandenen maximalen strukturellen Übereinstimmungen verbinden. Schließlich haben $HTH_\alpha$ und $HTH_\beta$ bzw. H2B nahe am Ende der in Fig. 3(A) gezeigten Bereiche Lys-Arg-Paare und andere dibasische Strukturen, die als typische Signale für Proteasen gelten, die aktive Hormone aus ihren Proformen freisetzen. Solche Signalstellen in Histonen sind für die Lokalisierung der erfindungsgemäß wichtigen aktiven Sequenzen mit immunstimulierenden und/oder endokrinologischen Eigenschaften von wesentlicher Bedeutung.

Schlußfolgerungen aus der Reinigung und Charakterisierung von HTH

HTH-Präparate enthalten zwei Komponenten, die konstant bei SDS-Polyacrylamidgel-Elektrophorese beobachtet wurden. Diese zwei Komponenten sind in hoher Ausbeute vorhanden und stimmen mit den Hormonaktivitäten der Präparate über- ein. Ändere Komponenten konnten ebenfalls bestimmt werden, aber in geringer und variabler Ausbeute und verschieden von Präparation zu Präparation. Die beiden Hauptkomponenten, $HTH_\alpha$ und $HTH_\beta$, wurden weiter geeinigt durch HPLC-Chromatographie. Wenn HTH kein Spurenbestandteil ist, sollten alle bisher für HTH beschriebenen biologischen Eigenschaften einer der beiden Komponenten zugeordnet werden oder aber auch beiden, wenn sie entweder synergistisch oder sich addierend agieren.

Die Strukturanalysen ergeben, daß $HTH_\alpha$ und $HTH_\beta$ 129 bzw. 125 Aminosäuren enthalten, was eine gute Übereinstimmung mit den durch SDS-Polyacrylamidgel-Elektrophorese geschätzten Molekulargewichtswerten von etwa 15000 ergibt. Diese Analysen zeigen ebenfalls, daß die Primärstrukturen von HTH$_\alpha$ und HTH$_\beta$ identisch den Histonen H2A bzw. H2B von Kalb und Mensch sind (23). Dies erklärt auch das für HTH durch Ausschlußchromatographie bestimmte Molekulargewicht von etwa 30000, da die inneren Histone in Form des dimeren H2A:H2B vorliegen. Keine Beweise für einen Äminosäureaustausch oder andere Modifikationen der Histonstrukturen konnten im HTH erhalten werden (23).

Mögliche Hormoneigenschaften und neuartige Funktionen der Histone.

Die Erkenntnis, daß HTH-Präparate die Histonpolypeptidketten H2A und H2B in offenbar dimere Konfiguration als Hauptbestandteile enthalten, läßt bisher unbekannte Möglichkeiten von neuartigen Funktionen für die Histone vermuten. Deshalb wurden die Histonstrukturen auf strukturelle Ähnlichkeiten mit anderen Thymushormonen, Histonen und in ihrer Funktion verwandten Molekülen hin untersucht. Da Peptidhormone häufig von größeren Proformen stammen, wurde dabei vor allem auf mögliche Signalstellen für Proteinspaltungen geachtet und außerdem auf Wechselwirkungen mit Histonmodifikationen und anderen Eigenschafte. Öbwohl die individuellen Beobactungen jeweils für sich allein nicht signifikant sind, werden sie im folgenden behandelt, und zwar um eine Zusammenfassung der neuen funktionellen Möglichkeiten zu erhalten.

Strukturelle Vergleiche

Die in den Fig. 2 und 3(A) aufgezeigten Ähnlichkeiten zeigen hohe Übereinstimmung zwischen den Histonen und den verglichenen Proteinen. Jeweils für sich selbst ist der Vergleich einzelner Paare kaum bedeutend (möglicherweise außer in Fig. 2), aber die kombinierten Ähnlichkeiten stimmen mit anderen Beobachtungen überein. Verwandte Bindungswechselwirkungen (29) und andere funktionelle Eigenschaften mögen genügen, um die Ähnlichkeiten zu erklären. Im Verhältnis zu der Identität zwischen HTH und den Histonen H2 und zu früheren Vergleichen (29) zeigen die strukturellen Übereinstimmungen in Fig. 3(A), daß die zwei Histone selbst eine Form von Prohormonen darstellen. Die Strukturen in Fig. 3(A) sind außerdem vereinbar mit der Existenz von Proformen der Thymosine und Thymopoietine, was durch die Existanz einer Vorstufe des Thymosin $\alpha_1$ (20) bekräftigt wird.

Prohormon-Analogien

Zwei Bereiche im H2B sind solchen Strukturen ähnlich, die typisch sind für Spaltungsstellen von Prohormonen, z.B. eine dibasische Aminosäuresequenz meistens in der Zusammensetzung Lys-Arg (21). Eine solche Region ist Lys-Arg-Lys-Arg beginnend auf Position 28, und eine andere ist Lys-Arg beginnend auf Position 85. Bei beiden fehlt ist Lys-Arg beginnend auf Position 85. Bei beiden fehlt ein folgendes Pro-Antisignal (22). Beide Positionen sind im Bereich von β-turns der

Sekundärstruktur außerhalb einer α-Helix vorausgesagt (30) und besitzen deshalb offenbar keine Stabilisierung gegen eine proteolytische Spaltung (31, 32). Dibasische Strukturen an ähnlichen Positionen sind im H2A ebenfalls vorhanden. Der erste Bereich bei H2A und H2B ist jeweils etwa am Ende der strukturellen Übereinstimmungen zwischen Histonen und Thymushormonen (Fig. 3(A)). Der zweite Bereich ist im C-terminalen Teil, der in Anlehnung an Fig. 3(A) mit der Region des Prealbumins übereinstimmt, für die früher vorgeschlagen wurde, daß sie den Spaltstellen in gastrointestinalen Hormonen entspricht (18).

Es zeigte sich, daß die Histone H2A und H2B in vitro ohne vorhergehende Denaturierung fur eine begrenzte Zahl von Spaltungen zulassen. H2B wird hauptsächlich nach Lys-20 und Lys-23 (33) und H2A wird hauptsächlich nach Arg-11 und Lys-118 (34) gespalten. Folglich schützen die nativen Konformationen die Histonmoleküle gegen eine vollständige Proteolyse in einer Art, die ähnlich ist den selektiven Spaltungen in Prohormonen. Es wurden endogene Proteasen gezeigt, die in den gleichen N- und C-terminalen Regionen spalten (35, 36), und die entsprechenden freigesetzten Fragmente von H2B hatten ein Molekulargewicht von ungefähr 2000, was der ursprünglich für das HTH beschriebenen Größe entspricht. Hieraus wird erfinderseits gefolgert, daß der thymushormonale aktive Teil des HTH$_\beta$/H2B in der N-terminalen Region liegt (Fig. 3(A)), was für die erfindungsgemäße Verwendung bedeutsam ist. Die Existenz von Thymushormonvorläufern wird ebenfalls unterstützt durch die Lys-Arg-Struktur am C-Terminus des Thymopoietins. Aus den vorstehenden Ausführungen wird deutlich, daß die Spaltstellen der Histone für die Lokalisierung der biologisch aktiven Sequenzen erfindungsgemäß bedeutsam sind.

Chemische Modifikationen

Von den Histonen weiß man, daß sie in chemisch modifizierten Formen vorkommen (37). Serin-6 des Histon 28 wird in vivo leicht phosphoryliert, und Ser-32 und Ser-36 können wahrscheinlich in vitro phosphoryliert werden durch cAMP-abhängige Proteinkinase(n) (38). Diese beiden Phosphorylierungsstellen liegen in dem bereits besprochenen, erfindungsgemäß wesentlichen N-terminalen Histon-Bereich. Erfinderseite wird deshalb gefolgert, daß die Phosphorylierung/Dephosphorylierung nicht nur in die Regulierung der konformativen Eigenschaften der Histone im Chromatin, sondern auch in die Signalregulierung an Stellen mit einbezogen ist, die für Spaltungen empfindlich sind.

Weitere Folgerungen in funktioneller Hinsicht

Etwa 5—15% des H2A und einige wenige Prozent des H2B sind an Ubiquitin (39, 40) gebunden. Eine Beziehung zwischen der Bindung von Ubiquitin en Proteine und die Regulierung des Proteinabbaus wurde beabachtet (15). Abgesehen von den angenommenen Funktionen des Ubiquitins bei der Chromatinkondensation oder bei der Genexpression im Nucleus könnte deshalb das Ubiquitin auch als ein Signal für das Auffalten und die folgende selektive Spaltung des HTH bzw. des H2A, H2B fungieren. Da das Ubiquitin in den Nuclei nahezu aller Zelltypen zu finden ist, sind möglicherweise für das Thymusgewebe spezifische Proteasen und chemische Modifikationen notwendig für die Spaltungen und Aktivitäten des HTH (H2A, H2B). Ein Einfluß auf den Proteinkatabolismus und die Ubiquitinfunktionen würde ebenfalls eine direkte Beziehung mit mehreren Effekten des HTH (H2A, H2B) auf des regulierende System zeigen.

Erfinderseits konnten damit erstmals zusätzliche Funktionen des H2A und H2B, außer ihren Eigenschaften als Strukturproteine des Chromatins, aufgezeigt werden, und zwar aufgrund ihrer bedeutend höheren Bindungs- und Abbauraten im Vergleich zu H3, H4 und DNA (41) und aufgrund einer möglichen totalen Rekonstitution der nativen core-Histonzusammensetzung nach Entfernung des H2A und H2B (42).

Wechselbeziehungen mit einer Bindungsregion Die Homologie eines Bereichs im Thymopoietin mit den Bindungsregionen von Molekülen mit einer ähnlich den Immunglobulinen gefalteten Struktur ist bereits vorgeschlagen worden (29). Dieses frühere Vergleich wurde erfinderseits unter Verwendung der vorhandenen Strukturen (Fig. 3(A) und 3(B)) erweitert. Erfinderseits wurde gefunden, daß für H2B dieser Bereich eine interne Wiederholung (Region 16—20 und 116—220 sind identisch) in derselben Art enthält, wie Immunglobulinstrukturen sich mit etwa dem gleichen Abstand wiederholen. Eine solche strukturelle Ähnlichkeit ist auch vareinbar mit den bekannten Kreuzreaktionen der Antihiston-Antikörper mit dem Fc-Abschnitt der·schweren Ketten der Immunglobuline (43) oder mit einem Zelloberflächenprotein auf menschlichen Leukozyten (44).

Schlußfolgerungen

Aufgrund der erfinderseits aufgezeigten Übereinstimmungen zwischen aktiven Eigenschaften des HTH und der Histone H2A und H2B können jetzt bisher unbekannte Funktionen der Histone erklärt werden. Die erfinderseitigen Beobachtungen von Thymushormon/Histon-Ähnlichkeiten lassen auf neue Wechselbeziehungen mit Ubiquitin schließen, die möglicherweise einen molekularen Mechanismus für die HTH/Histon-Funktion bei der Regulierung des Proteinabbaus andeuten.

Schließlich erlauben die erfinderseitigen Beobachtungen Rückschlusse auf die Evolution. Eine angestammte Verbindung zwischen den zu dem betrachteten Bereich gehörenden Molekülen könnte eine Erklärung für die beobachteten Ähnlichkeiten sein, die sich auf frühere Vorschläge einer "Überfamilie" (43) ausweiten läßt. De es Anzeigen für bevorzugte Bindungsregionen gibt und biologische Aktivitäten von HTH/Histonen durch Ubiquitin oder andere gewöhnliche Proteine freigesetzt werden könnten, werden die Ähnlichkeiten durch konvergierende Evolution erklärbar. Was auch immer der Ursprung dieser erfinderseits erkannten verwandtschaftlichen Beziehungen ist, so liefern sie doch neue Möglichkeiten zum Verständnis der Wechselwirkungen zwischen HTH, Histonen und Ubiquitin und damit zur Schaffung von erfindungsgemäßen biologischen Wirksubstanzen mit thymushormonartigen Eigenschaften bzw. zur Angabe erfindungsgemäßer Verfahren zur Herstellung solcher Wirksubstanzen und zur Verwendung von Histonen oder deren aktiven Sequenzen in medizinischen bzw. pharmazeutischen Präparaten mit standardisierter Präparatwirkung.

Wasentliche Teile der Anmeldung sind zur Veröffentlichung von den Erfindern in Proceedings of the National Academy of Sciences, U.S.A., angemeldet.

Literaturverzeichnis

1. Bezssonoff, H. A. % Comsa. J. (1958) Ann. Endocr. 19. 222—227.

2. Bernardi, G. & Comsa. J. (1965) Experientia 21, 416—417.

3. Comsa. J. (1965) Am. J. Med. Sci. 250, 79—85.

4. Comsa. J. & Filipp. G. (1966) Ann. Instit. Pasteur 110. 365—372.

5. Comsa. J. (1973) in Thymic Hormones, ed. Luckey T. D., (University Press, Baltimore), pp. 39—58.

6. Comsa. J. (1973) in Thymic Hormones. ed. Luckey. T. D., (University Press. Baltimore). pp. 59—96.

7. Comsa. J. Leonhardt, H. & Wekerle, H. (1982) Rev. Physiol, Biochem. Pharmacol, 92, 115—191.

8. Comsa. J., Baumann, B., Zeppezauer, H., Leonhardt, H. & Weber, H. (1979) C. W. Acad. Sc. Paris 288, 185—187.

9. Low, T. L. K. & Goldstein, A. L. (1979) J. Biol. Chem. 254, 987—995.

10. Caldarella, J. Goodall, G. J., Felix, A. H., Reimer, E. P., Salvin, S. B. & Horecher, B. L. (1983) Proc, Natl., Acad. Sci. USA 80, 7424—7427.

11. Low, T. L. K., Hu, S.-K. & Goldstein, A. L. (1981) Proc. Natl. Acad. Sci. USA 78, 1162—1166.

12. Hannappel, E., Davoust, S. & Horecker, B. L. (1982) Proc. Natl. Acad. Sci. USA 79, 1708—1711.

13. Audhya, T., Schlesinger, D. H. & Goldstein, G. (1981) Biochemistry 28, 6195—6200.

14. Pleau, J.-M., Dardenne, H., Blouquit, Y. & Bach, J.-F. (1977) J. Biol. Chem. 252, 8045—8047.

15. Hershko, A. (1983) Cell 34, 11—12.

16. Schlesinger, D. H., Goldstein, (G. & Hiall, W. D. (1975) Biochemistry 14, 2214—2218.

17. Burton, P., Iden. S., Mitchell. K. & White, A. (1978) Proc. Natl. Acad. Sci. USA 75, 823—827.

18. Jörnvall, H., Carlström, A., Pettersson, T., Jacobsson, B., Persson, M. & Mutt, Y. (1981) Nature 291, 261—263.

19. Robey, G., Campbell, B. J. & Luckey, T. D. (1972) Infection a. Immunity 6, 682—688.

20. Haritos, A. A., Goodall, G. J. & Horecker, D.

L. (1984) Proc. Natl. Acad. Sci. USA 81, 1008—1011.

21. Pradayrol, L., Jörnvall, H., Mutt. V. & Ribet. A. (1980) FEBS Lett. 109, 55—58.

22. Jörnvall, H. & Persson, B. (1983) Biosci. Rep. 3. 225—232.

23. Rechhart, R., Jörnvall, H. & Zeppezauer, M. (1984) FEBS Lett., in Druck.

24. DeLange, R. J., Hooper, J. A. & Smith, E. L. (1972) Proc. Natl. Acad. Sci. USA 69, 882—884.

25. Olson, M. D. J., Jordan, J. & Busch, H. (1972) Biochem. Biophys. Res. Common. 46, 50—55.

26. Kanda, Y., Goodman, D. S., Canfield, R. E. & Morgan, F. J. (1974) J. Biol. Chem. 249, 6796—6805.

27. Jörnvall. H., Mutt, V. & Persson, M. (1982) Hoppe-Seyler's Z. Physiol. Chem. 363. 475—483.

28. Ball. D. J., Slaughter, C. A., Hensley, P. & Garrard, W. T. (1983) FEBS Lett. 154, 166—170.

29. Hahn, G. S. & Hamburger, R. N. (1981) J. Immunol, 126, 459—462.

30. Moss, T., Cary, D. P., Abercrombie, B. D., Crane-Robinson, C. & Bradbury, E. M. (1976) Eur, J. Biochem, 71, 337—350.

31. Geisow. M. J. & Smyth, D. G. (1980) in The Enzymology of Post-translational Modification of Proteins. Vol. 1 (Ed.: Freedman, R. B. & Hawkins, H.C.) Academic Press, London, pp. 259—287.

32. Jörnvall, M., Ekman. R., Carlquist, M. & Persson, B. (1984) in Biogenetics of Neurohormonal Peptides (Eds.: Håkanson, R. & Thorell. J.) Academic Press, London, in press.

33. Böhm. L., Briand, G., Sautière, P. & Crane-Robinson, C. (1982) Eur. J. Biochem. 123, 299—303.

34. Böhm, L., Crane-Robinson, C. & Sautière, P. (1980) Eur. J. Biochem. 106, 525—530.

35. Rill. R. L. & Oosterhof, D. K. (1982) J. Biol. Chem. 257, 14875—14880.

36. Eickbush, T. H., Watson, D. K. & Moudrianakis, E. N. (1976) Cell 9, 785—792.

37. Isenberg, I. (1979) Ann. Rev. Biochem. 48, 159—191.

38. Yeaman, S. J., Cohen, P., Watson, D. C. & Dixon, G. M. (1977) Biochem. J. 162, 411—421.

39. Busch, H., Ballal, N. R., Busch, R. K., Choi. Y. C., Davis, F., Goldknopf, I. L., Matsui, S. I. Rao, M. S. & Rothblum, L. J. (1978) Cold Spring Harbor Symp. Quant. Biol. 42, 665—683.

40. West, M. H. P. & Donner, W. M. (1980) Nucl. Acids Res. 8, 4671—4680.

41. Grove, G. W. & Zweidler, A., (1984) Biochemistry 23, 4436—4443.

42. Jordano, J., Montero, F. & Palacian, E. (1984) Biochemistry 23, 4280.

43. Hannestad, K. & Stollar, B. D. (1978) Nature 275, 671—673.

44. Rekvig. O. P. & Hannestad, K. (1980) J. Exp. Med. 152, 1720—1733.

45. Williams, A. F. (1984) Nature 308, 12—13.

## Patentansprüche

1. Wenigstens ein Histon und/oder wenigstens ein Histonabschnitt mit hormonellen Wirkungen zur Anwendung in therapeutischen Verfahren und in Diagnoseverfahren.

2. Histon nach Anspruch 1, dadurch gekennzeichnet, daß es sich um H2A und/oder H2B handelt.

3. Histon nach Anspruch 1, dadurch gekennzeichnet, daß es sich um H3 handelt.

4. Histon nach Anspruch 1, dadurch gekennzeichnet, daß der Histonabschnitt der evolutionär variable Abschnitt ist.

5. Histon nach Anspruch 1, dadurch gekennzeichnet, daß der Histonabschnitt der N-terminale Abschnitt ist.

6. Histon nach Anspruch 1, dadurch gekennzeichnet, daß es sich um wenigstens einen Histonabschnitt des H2A mit der Teilsequenz 1—41 oder 1—36 oder 1—12 oder 1—11 oder 12—36 oder 12—41 oder 1—28 oder 11—23 handelt.

7. Histon nach Anspruch 1, dadurch gekennzeichnet, daß es sich um wenigstens einen Histonabschnitt des Histonuntertyps H2A Z mit der Teilsequenz 1—30 oder 9—25 handelt.

8. Histon nach Anspruch 1, dadurch gekennzeichnet, daß es sich um wenigstens einen Histonabschnitt der Histons H2B mit der Teilsequenz 1—35 oder 1—34 oder 1—32 oder 1—31 oder 1—29 oder 1—26 oder 1—23 oder 24—35 oder 24—34 oder 24—32 oder 24—31 oder 24—29 oder 1—20 oder 21—29 oder 21—31 oder 21—32 oder 21—34 oder 21—35 oder 14—26 handelt.

9. Histon nach Anspruch 1, dadurch gekennzeichnet, daß es sich um einen Histonabschnitt des Histonuntertyps H2B P.A. mit der Teilsequenz 17—50 handelt.

10. Histon nach Anspruch 1, dadurch gekennzeichnet, daß es sich um einen Histonabschnitt des Histons H3 mit der Teilsequenz 3—34 oder 17—29 handelt.

11. Histon nach einem der Ansprüche 1 bis 10, zur Immuntherapie, zur Therapie von endokrinologischen Störungen und zur Krebstherapie.

12. Histon nach Anspruch 11 zur Therapie strahleninduzierter Leukämie.

13. Histon nach Anspruch 11 zur Dämpfung der Funktion der Nebennierenrinde.

14. Histon nach Anspruch 11 zur Dämpfung der Funktion der Schilddrüse.

15. Histon nach Anspruch 11 zur Dämpfung der Funktion der Gonaden.

16. Histon nach Anspruch 11 zur Unterstützung der Funktion der Hypophyse.

17. Histon nach Anspruch 11 zur Bekämpfung der Folgen des Fehlens der Thymusdrüse (Thymektomie).

## Revendications

1. Au moins une histone et/ou au moins un segment d'histone à actions hormonales, destinés à être utilisés dans des techniques thérapeutiques et de diagnostic.

2. Histone selon la revendication 1, caractérisée en ce qu'il s'agit de H2A et/ou H2B.

3. Histone selon la revendication 1, caractérisée en ce qu'il s'agit de H3.

4. Histone selon la revendication 1, caractérisée en ce que le segment d'histone est le segment variable par dévolution.

5. Histone selon la revendication 1, caractérisée en ce que le segment d'histone est le segment N-terminal.

6. Histone selon la revendication 1, caractérisée en ce qu'il s'agit d'au moins un segment d'histone H2A comportant la séquence partielle 1—41 ou 1—36 ou 1—12 ou 1—11 ou 12—36 ou 12—41 ou 1—28 ou 11—23.

7. Histone selon la revendication 1, caractérisée en ce qu'il s'agit d'au moins un segment d'histone du sous-ordre d'histone H2A Z, comportant la séquence partielle 1—30 ou 9—25.

8. Histone selon la revendication 1, caractérisée en ce qu'il s'agit d'au moins un segment de l'histone H2B, comportant la séquence partielle 1—35 ou 1—34 ou 1—32 ou 1—31 ou 1—29 ou 1—26 ou 1—23 ou 24—35 ou 24—34 ou 24—32 ou 24—31 ou 24—29 ou 1—20 ou 21—29 ou 21—31 ou 21—32 ou 21—34 ou 21—35 ou 14—26.

9. Histone selon la revendication 1, caractérisée en ce qu'il s'agit de segment du sous-type d'histone H2B P.A. comportant la séquence partielle 17—50.

10. Histone selon la revendication 1, caractérisée en ce qu'il s'agit d'un segment de l'histone H3, comportant la séquence partielle 3—34 ou 17—29.

11. Histone selon l'une quelconque des revendications 1 à 10, pour utilization en immunothérapie, dans la thérapie des troubles endocriniens et dans la thérapie du cancer.

12. Histone selon la revendication 11, pour utilisation dans la thérapie des leucémies provoquées par irradiation.

13. Histone selon la revendication 11, utilisée pour atténuer la fonction des corticosurrénales.

14. Histone selon la revendication 11, utilisée pour atténuer la fonction de la glande thyroïde.

15. Histone selon la revendication 11, utilisée pour atténuer la fonction des gonades.

16. Histone selon la revendication 11, utilisée pour soutenir la fonction de l'hypophyse.

17. Histone selon la revendication 11, utilisée pour combattre les conséquences d'une absence de thymus (thymectomie).

## Claims

1. At least one histone and/or at least one histone segment having hormonal activity for use in therapeutic and diagnostic procedures.

2. A histone according to claim 1, characterised in that it is histone H2A and/or histone H2B.

3. A histone according to claim 1, characterized in that it is histone H3.

4. A histone according to claim 1, characterized in that the histone segment is the evolutionary variable part.

5. A histone according to claim 1, characterized in that the histone segment is the N-terminal part.

6. A histone according to claim 1, characterized in that it is at least one histone segment of the partial sequences 1—41 or 1—36 or 1—12 or 1—11 or 12—36 or 12—41 or 1—28 or 11—23 of histone H2A.

7. A histone according to claim 1, characterized in that it is at least one histone segment of the partial sequences 1—30 or 9—25 of histone subtype H2A Z.

8. A histone according to claim 1, characterized in that it is at least one histone segment of the partial sequences 1—35 or 1—34 or 1—32 or 1—31 or 1—29 or 1—26 or 1—23 or 24—35 or 24—34 or 24—32 or 24—31 or 24—29 or 1—20 or 21—29 or 21—31 or 21—32 or 21—34 or 21—35 or 14—26 of histone H2B.

9. A histone according to claim 1, characterized in that it is a histone segment of the partial sequence 17—50 of histone subtype H2B P.A.

10. A histone according to claim 1, characterized in that it is a histone segment of the partial sequences 3—34 or 17—29 of histone H3.

11. A histone according to one of the claims 1 to 10, for immunotherapy, the therapy of endocrine malfunctions and cancer therapy.

12. A histone according to claim 11, for the therapy of radiation-induced leukaemia.

13. A histone according to claim 11, for suppressing the function of the adrenal cortex.

14. A histone according to claim 11, for suppressing the function of the thyroid gland.

15. A histone according to claim 11, for reducing the function of the gonads.

16. A histone according to claim 11, for supporting the function of the pituitary.

17. A histone according to claim 11, for controlling the consequences of the absence of the thymus gland (thymectomy).

Fig. 1A

ABSORBANZ bei 214nm (——)

%B (————)

VOLUMEN (mℓ)

Fig. 1B

HTH$_\beta$ = H2B

HTH$_\alpha$ = H2A

ABSORBANZ bei 214nm (——)

%B (————)

VOLUMEN (mℓ)

## Fig. 2

```
                        70                    80                      90
H2A  I L E L A G N A A R D N K K T R I I P R H L Q L A I R N D E E L N
                        80                    90                     100
H2B  I A G E A S R L A H Y N K R S T I T S R E I Q T A V R L L L P G E
                       110                   120                     130
H3   L F E D T N L C A I H A K R V T I M P K D I E L A R R I R G E R A
```

EP 0 149 468 B1

## Fig. 3A

| Proteine | Segmente | Aminosäuresequenzen |
|---|---|---|
| H3 | 3-34 | T K Q T A R K S - T G G K A P - - R K Q L A T K A - - - A R K S A P A T G G |
| H2B | 1-31 | P E P A - K S A P A P K - - K G S K K A V T K A - - - Q K K D G K R K R |
| H2B P.A. | 17-50 | T K R S P Q K G G K G G K G A K R G G K A G - K R - - - R R G V Q V K R R R |
| H2A | 1-28 | Ac S G R G K Q G G K A - - - R A K A K T R S - - - S R A G L Q F P V G |
| H2A.Z. | 1-30 | A G G K A G K D S G K A K T K A V S X Q R A G L Q F P V G |
| FTS | 1-9 | E A - K S - Q G G S N |
| Thymopoietin | 6-40 | D P S V L T K - E K L K S - E L V - A N H V T L P A G E Q R K D V Y V E L Y |
| Thymosin α11 | 2-35 | D A A V D T S S E I T T K D L K E K K E V V E E A - E N G R E A P A N |
| Thymosin β4 | 10-42 | E K - F D - K S - K L K K T E T - Q E K N - P L P S K E T I E Q E K Q A G E |
| Thymosin β9 | 9-41 | I N S F D - K A - K L K K T E T - Q E K N - T L P T K E T I E Q E K Q A K |
| Ubiquitin | 1-27 | M Q I F V K T L T - - G K T I T L - E V E - P S D T I E N V K |
| Prealbumin | 34-70 | R K A A D Q T W E P U G K T S E S G E L H G - L T T Z Z Q F V - E G I Y K V |

## Fig. 3B

| Proteine | Segmente | Aminosäuresequenzen |
|---|---|---|
| H3 | 17-29 | R K Q L A T K A - - - A R K S A |
| H2B | 14-26 | S K K A V T K A - - - Q K K D G |
| H2A | 11-23 | R A K A K T R S - - - S R A G L |
| H2A.Z. | 9-25 | S G K A K T K A V S X Q R A G L |
| Thymopoietin | 21-35 | - A N H V T L P A G E Q R K D V |
| Thymosin α11 | 19-33 | K K E V V E E A - E N G R E A P |
| Thymosin β9 | 23-37 | Q E K N - T L P T K E T I E Q E |
| Ubiquitin | 10-22 | - G K T I T L - E V E - P S D T |
| Thy-1 | 66-80 | F I K V L T L A - N F T T K D E |
| IgE | 497-511 | S R L E V T R A - E W Q E K D E |
| IgG | 408-422 | S K L S V P T S - E W Q R G D V |
| HLA | 245-259 | A A V V P S G - E E Q R Y T C |
| β2-m | 64-78 | L L Y S Y T E F - T P T E K D E |

3